# EUROPEAN PATENT APPLICATION

(11) **EP 4 455 664 A1**
(43) Date of publication of application: **30.10.2024**
(21) Application number: 22912037.3
(22) Date of filing: 23.12.2022
(51) Int. Cl.: G01N 33/50, A61P 35/00

(54) **COMPOSITION COMPRISING TAF10 INHIBITOR FOR PREVENTING OR TREATING COLORECTAL CANCER**

(30) Priority: 23.12.2021 KR 20210185905
(71) Applicant: Korea Advanced Institute of Science and Technology, Daejeon 34141 (KR)
(72) Inventor: CHO, Kwang Hyun, Daejeon 34141 (KR); LEE, Hye Min, Daejeon 34141 (KR); GONG, Jeong Ryeol, Daejeon 34141 (KR)
(74) Representative: Potter Clarkson
(86) International application number: PCT/KR2022/021228
(87) International publication number: WO 2023/121404

(57) **Abstract**

The present invention relates to a composition comprising a TAF10 inhibitor for preventing or treating colorectal cancer. The inhibition of TAF10 was found to lead to suppressing the growth and proliferation of colorectal cancer cells, reducing vascularization, and inhibiting the WNT signaling pathway, and increase the survival rate of patients as analyzed with a large-scale cancer database. In particular, reversal of the cancerization process, which is the differentiation of colorectal cancer cells into normal cells, was confirmed. Unlike conventional anticancer agents that simply induce the death of cancer cells, the present invention can thus be advantageously utilized as a treatment method for converting colorectal cancer cells into normal cells while excluding side effects of normal cell death that may occur during anticancer treatment.

## Description

### [Technical Field]

The present invention relates to a composition including a TAF10 inhibitor for preventing or treating colorectal cancer.

### [Background Art]

Cancer is one of incurable diseases that humanity has to solve, and a huge amount of capital is being invested in development to cure the cancer around the world. In Korea, the cancer is the number one cause of death due to disease, in which about 100,000 or more people are diagnosed every year, and about 60,000 or more people have died. Carcinogens as cause factors of such cancer include smoking, ultraviolet rays, chemicals, food, and other environmental factors. However, the causes are diverse, so that it is difficult to develop therapeutic agents and the effectiveness of the therapeutic agents is also different depending on an occurring site. Substances currently used as the therapeutic agents have significant toxicity and do not selectively remove cancer cells, so that there is an urgent need to develop less toxic and effective anticancer agents to not only treat cancer after development, but also prevent the development of cancer.

Cancer is broadly classified into blood cancer and solid cancer, and occurs in almost all regions of the body, including lung cancer, gastric cancer, breast cancer, colorectal cancer, oral cancer, liver cancer, uterine cancer, esophageal cancer, and skin cancer. As a result of calculating national cancer incidence statistics, cancer with the highest increase in mortality compared to 1996 is lung cancer, followed by colorectal cancer, prostate cancer, and pancreatic cancer. In particular, colorectal cancer is one of the most common cancers worldwide, and the incidence of colorectal cancer in Korea is 12.0% of the entire cancer incidence, ranking 3rd and 4th in cancer incidence and mortality by organ, respectively, and is gradually increasing. Among the major cancers in men, the greatest cancers increased in incidence were prostate cancer and colorectal cancer, and age-standardized incidence rates increased by 74.1% and 50.4%, respectively, in 2005 compared to 1999. Considering it has been reported in developed countries, the three most common types of cancer are representatively prostate cancer, colorectal cancer, and lung cancer in men, and breast cancer, colorectal cancer, and lung cancer in women, even in Korea, where lifestyles are gradually becoming westernized, it is expected that an increase in colon cancer, prostate cancer, and breast cancer will accelerate. Currently, in the case of other diseases, the death rate is slowing down as treatment technology develops and people continue to manage the diseases. However, as the incidence of colorectal cancer is rapidly increasing, research on developing drugs to treat colorectal cancer is also actively increasing.

The primary treatment principle for colorectal cancer is surgical resection, but since there is a high recurrence rate even after surgical resection, auxiliary treatment such as radiotherapy or chemotherapy is required to prolong survival duration, alleviate symptoms, and maintain and improve the quality of life. However, there are no absolute principles regarding the type and route of administration of drugs in chemotherapy, and the effectiveness is also not satisfactory. In addition, even among patients with the same colorectal cancer, there are significant differences in response rate and survival probability to chemotherapy. To date, active research has been conducted on drugs targeting the tumor's genetic predisposition, particularly growth signaling transduction, and a microenvironment of tumor cells, to develop therapeutic agents for patients with solid cancers including colorectal cancer, but satisfactory therapeutic agents have not been developed.

In particular, it is known that the activity of epigenetic regulators is required along with the accumulation of mutations in the cancerization process of converting normal cells into cancer cells. Therefore, there is a need for the development of technology that shows excellent therapeutic activity against colorectal cancer and may differentiate into normal cells or normal-like cells.

### [Disclosure]

### [Technical Problem]

The present inventors have developed a technology capable of showing an excellent therapeutic effect on colorectal cancer and causing reversal of the cancerization process of colorectal cancer, and confirmed that a specific gene was suppressed to show an excellent therapeutic effect on colorectal cancer and induce differentiation of colorectal cancer cells into normal cells, and then completed the present invention.

An object of the present invention is to provide a pharmaceutical composition for treating or preventing colorectal cancer.

Another object of the present invention is to provide a food composition for preventing or alleviating colorectal cancer.

Yet another object of the present invention is to provide a pharmaceutical composition for enhancing sensitivity of colorectal cancer cells to anticancer agents.

Still another object of the present invention is to provide an anticancer adjuvant.

Still another object of the present invention is to provide a reagent composition for controlling differentiation of colorectal cancer cells into normal cells or normal-like cells.

Still another object of the present invention is to provide a method for inducing proliferation suppression or normal differentiation of colorectal cancer cells.

Still another object of the present invention is to provide a screening method of agents for treating colorectal cancer.

Still another object of the present invention is to provide a method for treating colorectal cancer.

### [Technical Solution]

One aspect of the present invention provides a pharmaceutical composition for preventing or treating colorectal cancer, including a TATA-Box Binding Protein Associated Factor 10 (TAF10) inhibitor.

Further, another aspect of the present invention provides a food composition for preventing or alleviating colorectal cancer, including a TATA-Box Binding Protein Associated Factor 10 (TAF10) inhibitor.

Further, yet another aspect of the present invention provides a pharmaceutical composition for enhancing sensitivity of colorectal cancer cells to anticancer agents, including a TATA-Box Binding Protein Associated Factor 10 (TAF10) inhibitor.

Further, still another aspect of the present invention provides an anticancer adjuvant including a TATA-Box Binding Protein Associated Factor 10 (TAF10) inhibitor.

Further, still another aspect of the present invention provides a reagent composition for controlling differentiation of colorectal cancer cells into normal cells or normal-like cells, including a TATA-Box Binding Protein Associated Factor 10 (TAF10) inhibitor.

Further, still another aspect of the present invention provides a method for inducing proliferation suppression or normal differentiation of colorectal cancer cells, including treating colorectal cancer cells with the reagent composition according to the present invention.

Further, still another aspect of the present invention provides a screening method of agents for treating colorectal cancer, including (a) treating colon cancer cells with a candidate substance; (b) measuring the expression level of TAF10 in the colon cancer cells treated with the candidate substance; and (c) screening the candidate substance as a preparation for treating colorectal cancer when the expression level of TAF10 is lower than that of a control group that is not treated with the candidate substance.

Further, still another aspect of the present invention provides a method of treating colorectal cancer, including administering a TATA-Box Binding Protein Associated Factor 10 (TAF10) inhibitor to a subject.

### [Advantageous Effects]

According to the present invention, the inhibition of TAF10 was found to lead to suppressing the growth and proliferation of colorectal cancer cells, reducing vascularization, and inhibiting the WNT signaling pathway, and increase the survival probability of patients as analyzed with a large-scale cancer database. In particular, reversal of the cancerization process, which is the differentiation of colorectal cancer cells into normal cells, was confirmed. Unlike conventional anticancer agents that simply induce the death of cancer cells, the present invention can thus be advantageously utilized as a treatment method for converting colorectal cancer cells into normal cells while excluding side effects of normal cell death that may occur during anticancer treatment.

### [Description of Drawings]

FIG. 1 illustrates results of performing single-cell sequencing of normal and tumor organoids from a colorectal cancer patient and discovering important epigenetic regulator targets in a cancerization process of colorectal cancer through data analysis.
FIG. 2A illustrates a result of confirming an effect of inhibition of TAF10 on the proliferation of colorectal cancer cells.
FIG. 2B illustrates a result of confirming overall survival probability when TAF10 is inhibited in The Cancer Genome Atlas (TCGA) as a large-scale cancer database.
FIG. 2C illustrates a result of confirming an association between expression of TAF10 and cancer stem cells.

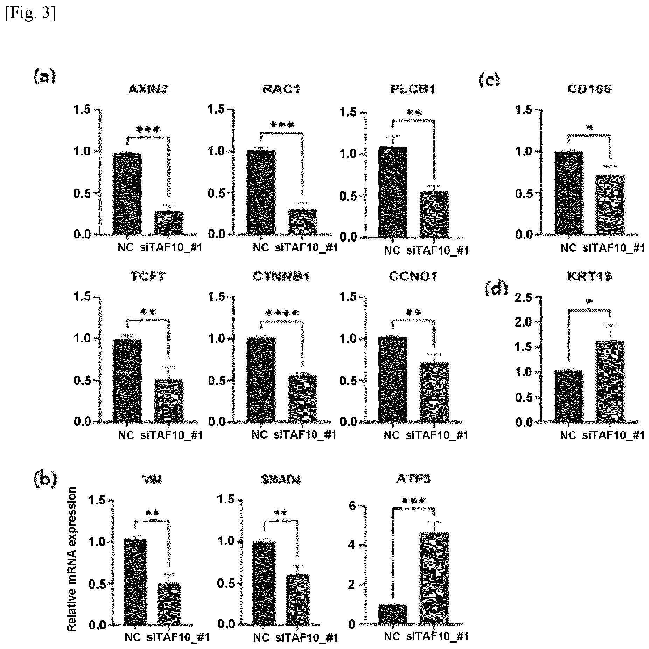

FIG. 3 illustrates results of confirming effects of TAF10 inhibition on WNT signaling pathway, metastatic potential, stemness, and expression of representative genes expressed by normal enterocytes. FIG. 3A illustrates a result of confirming the expression of genes of a WNT signaling pathway, FIG. 3B illustrates a result of confirming the expression of genes related to mesenchymal cells and tumor suppressor genes related to metastasis of colorectal cancer, FIG. 3C illustrates a result of confirming the expression of representative genes of sternness in the colon, and FIG. 3D illustrates a result of confirming the expression of representative genes of normal colorectal cells. In FIG. 3, * means P value < 0.05, ** means P value < 0.01, *** means P value < 0.001, and P value < 0.0001.
FIG. 4 illustrates results of confirming effects of TAF10 inhibition on different colorectal cancer cell lines. FIG. 4A illustrates a result of confirming the growth rate of colorectal cancer cell lines HT29 and CACO2 upon TAF10 inhibition and FIG. 4B illustrates a result of confirming the expression of genes and proteins related to cell growth.
FIG. 5A illustrates a result of confirming the expression of genes related to cell cycle arrest upon TAF10 inhibition in colorectal cancer cell lines HT29 and CACO2.
FIG. 5B illustrates a result of confirming cell cycles upon TAF10 inhibition in colorectal cancer cell lines HT29 and CACO2.
FIG. 5C illustrates a result of confirming the number of senescent cells upon TAF10 inhibition in colorectal cancer cell lines HT29 and CACO2. In FIG. 5C, a black bar represents 200 µM.
FIGS. 6A and 6B illustrate results of confirming cancer cell growth according to TAF10 inhibition in a colorectal cancer-inducing animal model. In FIG. 6A, a white bar represents 1 cm.
FIG. 6C illustrates a result of confirming survival probability according to TAF10 inhibition in a colorectal cancer-inducing animal model.
FIG. 7 illustrates results of confirming an effect of TAF10 inhibition on vascularization in a colorectal cancer-inducing animal model. FIG. 7A illustrates a result of confirming changes in vascularization upon TAF10 inhibition in a colorectal cancer-inducing animal model, FIG. 7B illustrates a result of confirming a regulatory pathway and a regulatory factor thereof, and FIG. 7C illustrates a result of confirming expression of vascular markers. In FIG. 7C, a yellow triangle indicates the expression of vascular markers.
FIG. 8A illustrates a result of comparing and analyzing bulk RNA sequencing data of a colorectal cancer cell line HCT16, which inhibits TAF10, and HCT16, which does not inhibit TAF10.
FIG. 8B illustrates a result of confirming changes in expression of genes or differentiation markers related to a WNT pathway based on the results of FIG. 8A.
FIG. 8C illustrates a result of visualizing bulk RNA sequencing data and TCGA patient data in a two-dimensional dot plot using sternness scores and differentiation scores in order to confirm a reversal effect at a transcriptome level by TAF10 inhibition.
FIG. 9A illustrates a result of confirming the confluence of cancer cells over time, as a result of confirming a synergistic effect of TAF10 inhibition and anticancer agents.
FIG. 9B illustrates an image result of cell death in each experimental group, as the result of confirming the synergistic effect of TAF10 inhibition and anticancer agents. In FIG. 9B, a white bar represents 200 µM.
FIG. 9C illustrates a result of confirming the degree of cell death using FACS analysis, as the result of confirming the synergistic effect of TAF10 inhibition and anticancer agents.
FIG. 9D illustrates a result of confirming a cancer volume over time, as the result of confirming the synergistic effect of TAF10 inhibition and anticancer agents. In FIG. 9D, *** means P value < 0.001.
FIG. 9E illustrates a result of a cancer size in each experimental group, as the result of confirming the synergistic effect of TAF10 inhibition and anticancer agents. In FIG. 9E, a white bar represents 1 cm.

### [Best Mode of the Invention]

Hereinafter, the present invention will be described in detail.

The present invention provides a pharmaceutical composition for preventing or treating colorectal cancer, including a TATA-Box Binding Protein Associated Factor 10 (TAF 10) inhibitor.

The TAF10 inhibitor may be at least one selected from the group consisting of siRNA, shRNA, miRNA, a ribozyme, an antisense nucleic acid, a DNA/RNA chimeric polynucleotide, an antibody, or a vector expressing these, targeting a TAF 10 gene or protein.

As used in the present invention, the term "antisense nucleic acid" refers to DNA, RNA, or derivatives thereof containing a nucleic acid sequence complementary to a specific mRNA sequence, and serves to inhibit the translation to a protein of mRNA by binding to the complementary sequence in mRNA. The antisense sequence refers to a DNA or RNA sequence complementary to mRNA of the gene and capable of binding to the mRNA, and may inhibit translation of the mRNA, translocation into the cytoplasm, maturation, or all other essential activities for overall biological functions.

In addition, the antisense nucleic acid may be modified at one or more base, sugar or backbone positions to enhance the efficacy. The nucleic acid backbone may be modified with phosphorothioate, phosphotriester, methyl phosphonate, short-chain alkyl, cycloalkyl, short-chain heteroatomic, heterocyclic intersugar linkages, and the like. In addition, the antisense nucleic acid may include one or more substituted sugar moieties. The antisense nucleic acid may include modified bases. The modified bases include hypoxanthine, 6-methyladenine, 5-methylpyrimidine (particularly, 5-methylcytosine), 5-hydroxymethylcytosine (HMC), glycosyl HMC, gentobiosyl HMC, 2-aminoadenine, 2-thio uracil, 2-thiothymine, 5-bromouracil, 5-hydroxymethyluracil, 8-azaguanine, 7-deazaguanine, N6(6-aminohexyl)adenine, 2,6-diaminopurine, and the like. In addition, the antisense nucleic acid may chemically bind to one or more moieties or conjugates that improve the activity and cell adhesion of the antisense nucleic acid. The antisense nucleic acid includes fat-soluble moieties, such as cholesterol moiety, cholesteryl moiety, cholic acid, thioether, thiocholesterol, fatty chain, phospholipid, polyamine, polyethylene glycol chain, adamantane acetic acid, palmityl moiety, octadecylamine, hexylaminocarbonyl-oxycol esterol moiety, and the like, but is not limited thereto. The antisense oligonucleotide may be synthesized in vitro by a conventional method to be administered in vivo, or may be synthesized in vivo.

As used in the present invention, "siRNA" means a nucleic acid molecule capable of mediating RNA interference or gene silencing. Since the siRNA may inhibit the expression of a target gene, the siRNA is provided as an efficient gene knock-down method or a gene therapy method.

The siRNA molecule of the present invention may have a structure in which a sense strand (a sequence corresponding to an mRNA sequence of a TAF 10 gene as a target gene according to an embodiment of the present invention) and an antisense strand (a complementary sequence to the mRNA sequence) are located at opposite sides each other to form a double chain, and the siRNA molecule of the present invention may have a single-stranded structure with self-complementary sense and antisense strands. Furthermore, siRNA is not limited to complete pairing of a doublestranded RNA portion paring RNAs, but may include a portion which is not paired by mismatch (corresponding bases are not complementary), bulge (there is no corresponding base on one chain), etc. In addition, when a siRNA end structure may suppress the expression of the target gene by an RNAi effect, both a blunt end and a cohesive end are possible, and the cohesive end structure is able to be both a 3'-end protruding structure and a 5'-end protruding structure.

The "shRNA" of the present invention is called small hairpin RNA or short hairpin RNA, and is used for silencing the gene by RNA interference. Usually, the shRNA is introduced into a target cell using a vector. Such a shRNA hairpin structure is also cleaved by other intracellular substances to become siRNA.

In the present invention, the siRNA included in the TAF10 inhibitor may include base sequences represented by SEQ ID NOs: 1 and 2, and the shRNA may include a base sequence represented by SEQ ID NO: 7, but are not limited thereto.

The present invention may include any one or more base sequences selected from the group consisting of SEQ ID NOs: 1, 2, and 7, and functional equivalents thereof. The "functional equivalent" refers to a polynucleotide that has sequence homology of at least 70% or more, preferably 80% or more, more preferably 90% or more, and even more preferably 95% or more with one or more base sequences selected from the group consisting of SEQ ID NOs: 1, 2, and 7 and exhibits substantially homogeneous physiological activity with a polynucleotide represented by one or more base sequences selected from the group consisting of SEQ ID NOs: 1, 2, and 7, as a result of deletion, substitution, or insertion of bases. The "% of sequence homology" with the polynucleotide is determined by comparing two optimally arranged sequences with a comparison region, and a part of a polynucleotide sequence in the comparison region may include addition or deletion (i.e., gap) compared to a reference sequence (without including addition or deletion) for an optimal alignment of the two sequences.

According to an example of the present invention, it was confirmed that when TAF 10 was inhibited in colorectal cancer cells, transcriptomes moved normally.

Therefore, in the pharmaceutical composition of the present invention, the TAF10 inhibitor is characterized by inducing differentiation of colorectal cancer cells into normal cells or normal-like cells.

In the present invention, the "normal-like cell" refers to a cell having a transcriptome of a cancer cell-derived normal cell, and refers to a cell with the same or similar activity as or to a normal cell, preferably a colorectal cell.

In addition, according to another example of the present invention, it was confirmed that when TAF10 is inhibited, the proliferation or growth of colorectal cancer cells was suppressed, and according to yet another example, it was confirmed that the vascularization in colorectal cancer tissue was reduced.

In addition, according to yet another example of the present invention, it was confirmed that when TAF10 was inhibited, the cancer sternness of colorectal cancer cells was reduced and senescence of colorectal cancer cells was accelerated.

Therefore, in the pharmaceutical composition of the present invention, the TAF10 inhibitor is characterized by suppressing the proliferation or growth of colorectal cancer cells. In addition, the TAF10 inhibitor is characterized by inhibiting the vascularization. In addition, the TAF10 inhibitor is characterized by accelerating the senescence of colorectal cancer cells.

In the present invention, the pharmaceutical composition may be characterized in the form of capsules, tablets, granules, injections, ointments, powders or beverages, and the pharmaceutical composition may be characterized by targeting humans. The pharmaceutical composition is not limited thereto, but may be formulated and used in the form of oral formulations, such as powders, granules, capsules, tablets, aqueous suspensions, etc., external preparations, suppositories, and sterile injectable solutions according to a conventional method, respectively.

The pharmaceutical composition according to the present invention may include a pharmaceutically acceptable carrier. The pharmaceutically acceptable carrier may be used with a binder, a lubricant, a disintegrant, an excipient, a solubilizer, a dispersant, a stabilizer, a suspending agent, a pigment, a flavoring, and the like during oral administration, may be mixed and used with a buffering agent, a preservative, a painless agent, a solubilizer, an isotonic agent, a stabilizer, and the like in the case of injections, and may be used with a base, an excipient, a lubricant, and a preservative, and the like in the case of topical administration. The formulations of the pharmaceutical composition of the present invention may be prepared variously in combination with the pharmaceutically acceptable carrier described above.

For example, for oral administration, the pharmaceutical composition may be formulated in the form of tablets, troches, capsules, elixirs, suspensions, syrups, wafers, etc., and for injections, the pharmaceutical composition may be formulated into a single dose ampoule or a multiple dose form. In addition, the pharmaceutical composition may be formulated into solutions, suspensions, tablets, capsules, sustained release agents, and the like.

Meanwhile, examples of the carrier, the excipient, and the diluent suitable for the formulations may be used with lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oils, or the like. In addition, the pharmaceutical composition may further include fillers, anti-coagulating agents, lubricants, wetting agents, flavorings, emulsifiers, preservatives, and the like.

The route of administration of the pharmaceutical composition according to the present invention is not limited thereto, but includes oral, intravenous, intramuscular, intraarterial, intramedullary, intrathecal, intracardiac, transdermal, subcutaneous, intraperitoneal, intranasal, intestinal, topical, sublingual or rectal administration. The "parenteral" includes subcutaneous, intradermal, intravenous, intramuscular, intraarticular, intrasynovial, intrasternal, intrathecal, intralesional and intracranial injection or infusion techniques. The pharmaceutical composition according to the present invention may also be administered in the form of a suppository for rectal administration.

The pharmaceutical composition according to the present invention may be variously changed according to various factors, including the activity of a specific active ingredient used, age, body weight, general health, sex, diet, administration time, administration route, excretion rate, drug combination, and the severity of a specific disease to be prevented or treated. The dose of the pharmaceutical composition varies depending on the condition, body weight, degree of a disease, drug form, and administration route and period of a patient, but may be appropriately selected by those skilled in the art. Preferably, the pharmaceutical composition may be administered with an amount capable of obtaining a maximum effect with a minimum amount without side effects in consideration of all the factors, and may be repeatedly administered in an effective dose of more preferably 1 to 10000 µg/weight kg/day, and much more preferably 10 to 1000 mg/weight kg/day several times a day. The dose does not limit the scope of the present invention in any aspect.

Further, the present invention provides a food composition for preventing or alleviating colorectal cancer, including a TATA-Box Binding Protein Associated Factor 10 (TAF 10) inhibitor.

The TAF10 inhibitor may be at least one selected from the group consisting of siRNA, shRNA, miRNA, a ribozyme, an antisense nucleic acid, a DNA/RNA chimeric polynucleotide, an antibody, or a vector expressing these, targeting a TAF 10 gene or protein.

In the present invention, the siRNA included in the TAF10 inhibitor may include base sequences represented by SEQ ID NOs: 1 and 2, and the shRNA may include a base sequence represented by SEQ ID NO: 7, but are not limited thereto.

The food composition according to the present invention includes all forms, such as functional food, nutritional supplements, health food, health supplements, and food additives. The type of food composition may be formulated in any one form selected from the group consisting of powders, tablets, capsules, pills, and liquids according to a conventional method known in the art, but is not limited thereto. The food composition may be formulated in various forms using methods known in the art.

For example, as the health food, the TAF10 inhibitor itself of the present invention may be ingested by granulation, encapsulation and powder or prepared in the form of tea, juice, and drinks to be drunk. In addition, the TAF 10 inhibitor of the present invention may be mixed with a substance or active ingredient known to have prevention, alleviation, or treatment activity of colorectal cancer to be prepared in the form of a composition.

In addition, the functional food may be prepared by adding the TAF 10 inhibitor of the present invention to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc.

In addition, the food composition of the present invention may include conventional food additives, and the suitability as the "food additive" is determined by the specifications and standards for the corresponding item in accordance with the general rules of the Food Additive Codex, general test methods, and the like approved by the Food and Drug Administration, unless otherwise specified. The items disclosed in the "Food Additives Codex" may include, for example, chemical composites such as ketones, glycine, calcium citrate, nicotinic acid, cinnamic acid, etc., natural additives such as persimmon color, licorice extract, crystal cellulose, Kaoliang color, guar gum, etc., and mixed formulations such as sodium L-glutamic acid formulations, alkali agents for noodles, preservative formulations, tar color formulations, etc.

In the food composition of the present invention, the TAF 10 inhibitor may be included preferably in an amount of 0.00001 to 50 wt% based on the food composition. If the content is less than 0.00001 wt%, the effect is insignificant, and if the content exceeds 50 wt%, an increase in effect compared to the amount used is minimal, which is uneconomical.

In addition, in order to use the TAF 10 inhibitor of the present invention in the form of food additives, the TAF 10 inhibitor may be prepared and used in the form of tablets, capsules, powders, granules, liquids, pills, etc.

When the composition of the present invention is prepared as beverages, like general beverages, the composition may include various flavoring agents or natural carbohydrates as an additional ingredient. The above-mentioned natural carbohydrates may be used with monosaccharides such as glucose and fructose, disaccharides such as maltose and sucrose, natural sweeteners such as dextrin and cyclodextrin, synthetic sweeteners such as saccharin and aspartame, and the like. A ratio of the natural carbohydrates may be generally about 0.01 to 10 g, preferably about 0.01 to 0.1 g per 100 ml of the composition of the present invention.

In addition, the composition of the present invention may include various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohols, a carbonic acid agent used in a carbonated drink, or the like. In addition, the composition of the present invention may include pulps for preparing natural fruit juices, fruit juice beverages or vegetable beverages. These ingredients may be used independently or in combination. Although the ratio of these additives is not greatly important, generally, the ratio thereof is selected in a range of 0.01 to 0.1 parts by weight per 100 parts by weight of the composition of the present invention.

As used in the present invention, the "health supplement" or "health functional food" refers to food prepared and processed using raw materials or ingredients with functionality, which are useful for the human body according to the Health Functional Foods Act. The "functionality" means ingestion for the purpose of adjusting nutrients for the structures and functions of the human body or obtaining useful effects on health applications such as physiological actions.

Meanwhile, according to an example of the present invention, it was confirmed that inhibition of TAF10 shows a synergistic effect on the anticancer effect of oxaliplatin.

Accordingly, the present invention provides a pharmaceutical composition for enhancing sensitivity of colorectal cancer cells to anticancer agents, including a TATA-Box Binding Protein Associated Factor 10 (TAF10) inhibitor.

The anticancer agent may include, without limitation, anticancer agents known in the art as long as the anticancer agent may enhance the effect of treating cancer or inhibiting cancer metastasis by combined treatment with the TAF10 inhibitor. For example, the anticancer agent may be at least one selected from the group consisting of oxaliplatin, 5-fluorouracil (5-FU), doxorubicin, irinotecan, carboplatin, paclitaxel, gemcitabine and bortezomib, but is not limited thereto. Preferably, the anticancer agent may be oxaliplatin.

The pharmaceutical composition for enhancing the sensitivity of colorectal cancer cells to the anticancer agent according to the present invention may be administered simultaneously or sequentially with the anticancer agent.

Details of the pharmaceutical composition for enhancing the sensitivity of colorectal cancer cells to the anticancer agent according to the present invention are the same as described for the pharmaceutical composition for preventing or treating colorectal cancer.

In addition, the present invention provides an anticancer adjuvant including a TATA-Box Binding Protein Associated Factor 10 (TAF10) inhibitor.

In the present invention, the "anticancer adjuvant" refers to an agent capable of alleviating, improving or enhancing an anticancer effect of the anticancer agent.

In the present invention, the anticancer adjuvant may be used as an anticancer agent or an anticancer adjuvant depending on a treatment concentration, and may enhance the sensitivity of the anticancer agent.

In the present invention, the anticancer adjuvant may be administered in combination with a known compound that has the effect of preventing, alleviating, or treating cancer. In this regard, the anticancer adjuvant may be administered simultaneously or sequentially with known compounds.

The known compound may be at least one anticancer agent selected from the group consisting of oxaliplatin, 5-fluorouracil (5-FU), doxorubicin, irinotecan, carboplatin, paclitaxel, gemcitabine and bortezomib, but is not limited thereto.

In addition, the present invention provides a reagent composition for controlling differentiation of colorectal cancer cells into normal cells or normal-like cells, including a TATA-Box Binding Protein Associated Factor 10 (TAF10) inhibitor.

In the reagent composition of the present invention, the TAF10 inhibitor is as described above.

According to an example of the present invention, it was confirmed that when TAF10 was inhibited in colorectal cancer cells, the transcriptomes migrated normally. Further, the present invention provides a method for inducing proliferation suppression or normal differentiation of colorectal cancer cells, including treating colorectal cancer cells with the reagent composition according to the present invention.

Further, the present invention provides a screening method of agents for treating colorectal cancer, including (a) treating colon cancer cells with a candidate substance; (b) measuring the expression level of TAF10 in the colon cancer cells treated with the candidate substance; and (c) screening the candidate substance as a preparation for treating colorectal cancer when the expression level of TAF10 is lower than that of a control group that is not treated with the candidate substance.

According to the screening method of the present invention, a candidate substance to be analyzed may first be in contact with cancer cells including the gene or protein. The candidate substance refers to an unknown substance used in screening to test whether the substance affects the expression level of the gene, the amount of protein, or the activity of the protein.

The candidate substance may include, but are not limited to, chemicals, antisense oligonucleotides, antisense-RNA, siRNA, shRNA, miRNA, antibodies specific for the protein, or natural product extracts.

In the present invention, the candidate substance may include siRNA, shRNA, miRNA, or antibodies specific for TAF 10.

Thereafter, the expression level of the gene, the amount of the protein, or the activity of the protein may be measured in the cells treated with the candidate substance. As the measured result, when it is measured that the expression level of the gene, the amount of the protein, or the activity of the protein is decreased, it may be determined that the candidate substance may be used as an agent capable of treating or preventing cancer.

The method of measuring the expression level of the gene or the amount of protein may be performed including a known process of isolating mRNA or protein from a biological sample using a known technique. The "biological sample" refers to a sample collected from a living body of which the gene expression level or protein level is different from that of the control group depending on the degree of occurrence or progression of cancer. Examples of the sample may include tissue, cells, blood, serum, plasma, saliva, and urine, but are not limited thereto.

The expression level of the gene is preferably measured by measuring the level of mRNA, and methods for measuring the mRNA level include reverse transcription polymerase chain reaction (RT-PCR), real-time reverse transcription polymerase chain reaction, RNase protection assay, Northern blot, DNA chips, etc., but are not limited thereto.

The protein level may be measured using an antibody. In this case, the marker protein in the biological sample and a specific antibody thereto form a combination, that is, an antigen-antibody complex, and the formed amount of the antigen-antibody complex may be measured quantitatively through the signal size of a detection label. The detection label may be selected from the group consisting of enzymes, fluorescent materials, ligands, luminescent materials, microparticles, redox molecules, and radioisotopes, but is not limited thereto. Analytical methods for measuring the protein levels include Western blot, ELISA, radioimmunoassay, radioimmunodiffusion, Ouchterony immunodiffusion, rocket immunoelectrophoresis, tissue immunostaining, immunoprecipitation assay, complement fixation assay, FACS, protein chips, etc., but are not limited thereto.

Further, the present invention provides a method of treating colorectal cancer, including administering a TATA-Box Binding Protein Associated Factor 10 (TAF10) inhibitor to a subject.

As used in the present invention, the term "subject" refers to an individual having a cancer disease, preferably mammals such as horse, sheep, pig, goat, dog, including humans having a cancer disease, but preferably human.

In the method of treating cancer of the present invention, the TAF10 inhibitor may be administered simultaneously or sequentially with the anticancer agent.

The above-described contents of the present invention are equally applied to each other unless otherwise contradict each other, and those appropriately modified and implemented by those skilled in the art are also included in the scope of the present invention.

Hereinafter, the present invention will be described in detail through Examples, but the scope of the present invention is not limited only to the Examples below.

### Experimental Example 1. Experimental method

### 1-1. Cell culture

Colorectal cancer cell lines HCT116, HT29, and CACO2 and a normal colon cell line NCM460 were used in cell experiments. The HCT116 was purchased from the Korean Cell Line Bank, and the HT29 and CACO2 were purchased from ATCC. The NCM460 was provided by KAIST. The cell lines were cultured in a DMEM supplemented with 10% FBS and antibiotics. All cells were cultured in an incubator at 5% CO₂ and 37°C.

### 1-2. Transfection and transduction of siRNA

Transfection reagents RNAiMAX (Thermo Fisher Scientific) and siRNA (BIONEER Corporation, Daejeon, South Korea) were mixed in an Opti-mem medium at a final concentration of 30 nM. Target cells were cultured with the mixture, and then cells were harvested for RNA extraction after 72 hours of siRNA transfection.

The used siRNAs siNPM1, siTAF10, and siZNHIT1 were as follows.
(SEQ ID NO: 1) siTAF10-SENSE: GUGAAGAAGCCGCACUACU
(SEQ ID NO: 2) siTAF10-ANTISENSE: AGUAGUGCGGCUUCUUCAC
(SEQ ID NO: 3) siNPM1-SENSE: GAAGCAGAGGCAAUGAAUUAC GA
(SEQ ID NO: 4) siNPM1-ANTISENSE: UCGUAAUUCAUUGCCUCUGCU UC
(SEQ ID NO: 5) siZNHIT1-SENSE: GAGACUGCCUCAGUUUGAU
(SEQ ID NO: 6) siZNHIT1-ANTISENSE: AUCAAACUGAGGCAGUCUC

### 1-3. Transfection and transduction of shRNA

According to the manufacturer's manual, shRNA targeting TAF10 (shTAF10: TRCN0000014603, Merck) and mixtures pLP1, pLP2, and pLP/VSVG for gene packaging were transfected to HEK293T cells using lipofectamine (Invitrogen, Waltham, USA) to produce lentivirus. Thereafter, HCT116 cells were transduced with 4 µg/ml of polybrene (Sigma-Aldrich). The infected cells were selected using 1 µg/ml of puromycin (Sigma-Aldrich).
(SEQ ID NO: 7) shTAF10: CCGCAAGTACACTCTAACCAT

### 1-4. Cell growth

Infected cells were inoculated in a 96-well plate (6 × 10³ cell/well) to measure the cell growth and the cell growth was recorded every 3 hours using IncuCyte ZOOM (Essen Bioscience), and cell confluence was analyzed using IncuCyte ZOOM 2016A software.

### 1-5. Total RNA extraction and qRT-PCR

Total RNA Extraction Kit (iNtRON Biotechnology, Gyeonggi, South Korea) was used to extract total RNA from cells. Total RNA was treated with RNase-free DNase I (Thermo Fisher Scientific) to prevent genomic DNA contamination. DNA was synthesized from total RNA using a DiaStar RT kit (Solgent, Daejeon, Korea) and a PCR system (Veriti 96-well Thermal Cycler; Applied Biosystems, Waltham, MA, United States) and quantitative real-time polymerase chain reaction (qRTPCR) was performed with a QuantStudio 5 real-time PCR system (Applied Biosystems) using the primers shown in Table 1.

**[Table 1]**

| **Target gene** | **Forward primer sequence (5'-3')** | **SEQ ID NO:** | **Reverse primer sequence (5'-3')** | **SEQ ID NO:** |
|---|---|---|---|---|
| TAF10 | CCACGCATAATTCGGCTCA T | 8 | CCTCCATGGTTAGGGTGTA CT | 9 |
| GAPDH | TGATGACATCAAGAAGGT GGTGAAG | 10 | TCCTTGGAGGCCATGTGGG CCAT | 11 |
| MKI67 | GAAAGAGTGGCAACCTGC CTTC | 12 | GCACCAAGTTTTACTACAT CTGCC | 13 |
| PCNA | CAAGTAATGTCGATAAAG AGGAGG | 14 | GTGTCACCGTTGAAGAGA GTGG | 15 |
| MCM7 | GCCAAGTCTCAGCTCCTGT CAT | 16 | CCTCTAAGGTCAGTTCTCC ACTC | 17 |
| TP53 | CCTCAGCATCTTATCCGAG TGG | 18 | TGGATGGTGGTACAGTCAG AGC | 19 |
| CDKN1A | AGGTGGACCTGGAGACTC TCAG | 20 | TCCTCTTGGAGAAGATCAG CCG | 21 |
| CXCL8 | GTGCAGTTTTGCCAAGGA GT | 22 | CTCTGCACCCAGTTTTCCT T | 23 |
| CTNNB1 | CACAAGCAGAGTGCTGAA GGTG | 24 | GATTCCTGAGAGTCCAAAG ACAG | 25 |
| CCND1 | TCTACACCGACAACTCCAT CCG | 26 | TCTGGCATTTTGGAGAGGA AGTG | 27 |
| TCF7 | GACATCAGCCAGAAGCAA G | 28 | CACCAGAACCTAGCATCA AG | 29 |
| AXIN2 | GAGGAGCGATCCTGTTAA TCC | 30 | GGTTCTCGGGAAATGAGGT AG | 31 |
| KRT19 | GCGAGCTAGAGGTGAAGA TC | 32 | CGGAAGTCATCTGCAGCCA | 33 |

### 1-6. Protein extraction and Western-blot

Cellular proteins were dissolved and extracted on ice in a lysis buffer (20 mM HEPES (pH 7.2), 150 mM NaCl, 0.5% Triton X-100, 10% glycerol, 0.1% SDS) containing 0.1% protease inhibitor and phosphatase inhibitor cocktail (Thermo-scientific, USA). The proteins were separated through protein electrophoresis (10% gel) and transferred to a PVDF membrane. For Western blot, an anti-TAF10 (ab263967) antibody was purchased from Abcam, anti-AKT1 (2938S), anti-phosphoAkt (2965S), anti-phospho B-raf (#2696), anti-MEK1/2. (#9122), anti-phosphoERK1/2 (#9106), anti-STAT3 (#9139), and anti-phosphoSTAT3 (#9131) antibodies were purchased from Cell signaling, and anti-phospho m-TOR (sc-293133), anti-phosphoMEK1/2 (sc-81503), anti-CyclinE1 (sc-247), anti-CDK2 (sc-6248), and anti-VEGF (sc7269) antibodies were purchased and used from Santacruz. A rabbit polyclonal anti-GAPDH antibody was provided and used from the Korea Research Institute of Bioscience and Biotechnology. The expression of each protein band was confirmed using an enhanced chemiluminescence (ECL) solution.

### 1-7. Fluorescence-activated cell sorting (FACS)

The cells were treated with trypsin-EDTA, separated into single cells, washed twice with PBS, and fixed with 70% ethanol for 1 hour at - 20°C. After washing again with PBS twice, the cells were treated with 20 µg/ml of RNase A, and flow cytometry analysis was performed through 20 µg/ml of propidium iodide (PI) staining.

### Experimental Example 2. Preparation of colorectal cancer-inducing animal model

First, TAF 10 WT (wild type) and TAF 10 knockdown (KD) cells were prepared in the same manner as in Experimental Example 1-3 using shRNA consisting of a base sequence represented by SEQ ID NO: 7 in HCT116, which was a colorectal cancer cell line. 2 × 10⁶ of TAF10 WT or TAF10 KD was prepared and subcutaneously injected into Nude mice deficient of an immune system. Tumor occurrence was confirmed every two days, and its size was measured. In addition, in order to confirm the effect of combined treatment with anticancer agents, the colorectal cancer-inducing animal model prepared in the same manner as before was divided into four experimental groups, and after 2 weeks (14 days) when the tumor had formed to some extent, a control group was administered with PBS and in the experimental group, oxaliplatin was injected into the abdominal cavity at a concentration of 10 mg/kg every two days.

### Example 1. Performance of single cell sequencing of normal and tumor organoids derived from colorectal cancer patients and discovery of epigenetic regulator targets

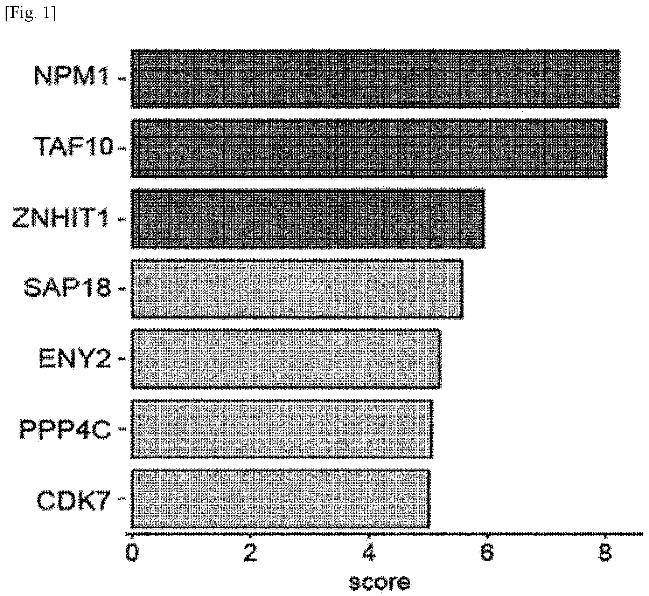

Single-cell sequencing of normal and tumor organoids from a colorectal cancer patient was performed and important epigenetic regulator targets were discovered in a cancerization process of colorectal cancer through data analysis. From the single cell sequencing results, single cell copy number variation (CNV) was inferred for each chromosomal loci using a COPYKAT package. Next, a phylogenetic tree starting from cells derived from the normal colon was constructed and it was confirmed that there was a separate branch in the process of metastasis from normal cells to cancer cells. In this branch, some cancer cells and normal cells were mixed, and in particular, cells with aneuploid mutations and polyploid cells were mixed, so that the cells corresponding to this branch were defined as cells in a metastatic state. Thereafter, a model capable of amplifying the number of single cells was constructed using a package called scDesign2 to secure a sufficient number of transition state cells. Meanwhile, a gene regulatory network (GRN) of 26 types of cancer tissues was downloaded from Bioconductor (https://bioconductor.org/packages/release/data/experiment/html/aracne.networks.ht ml) and then based on the GRN, the top three master epigenetic regulators NPM1, TAF10, and ZNHIT1 expected to regulate cells in the metastatic state were discovered using an ARACNe-metaviper algorithm, as illustrated in FIG. 1.

### Example 2. Selection of epigenetic regulator targets having the greatest effect on proliferation of colorectal cancer cell lines

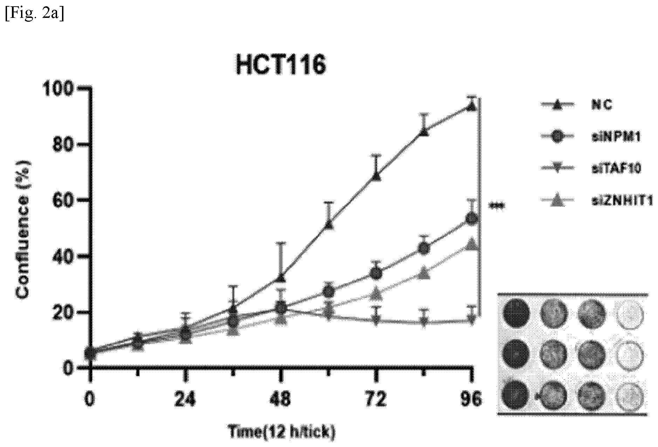

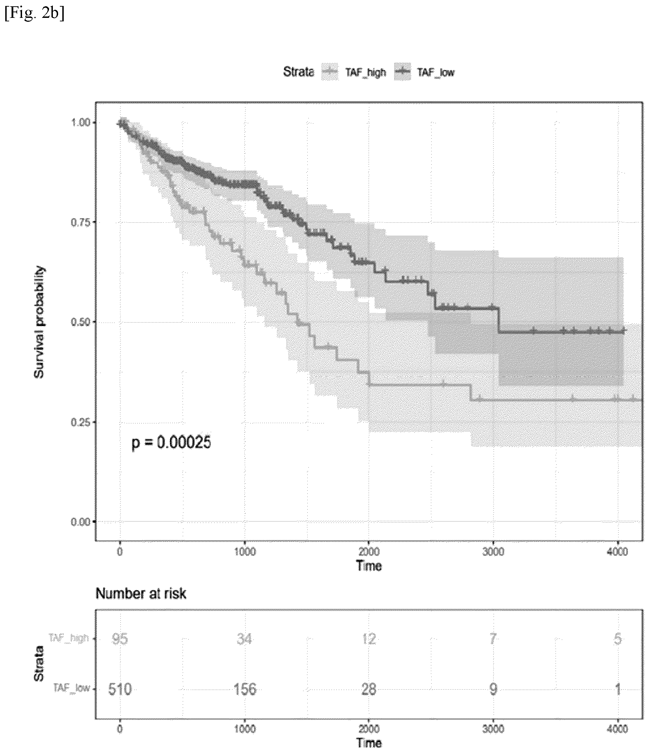

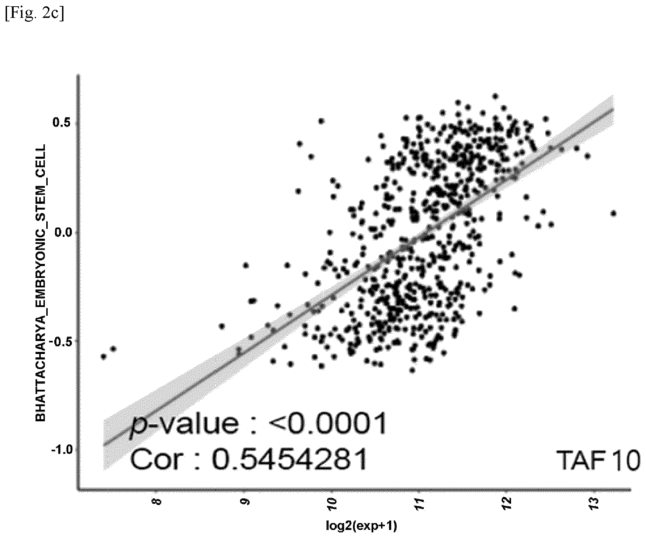

Based on those confirmed in Example 1, NPM1, TAF, or ZNHIT1 of the colorectal cancer cell line HCT116 was inhibited as in Experimental Example 1-2, and its growth ability was confirmed. As a result, as illustrated in FIG. 2, it was confirmed that in the colorectal cancer cell line, when TAF10 was inhibited, cell proliferation was significantly reduced (see FIG. 2A). In addition, as a result of confirming overall survival probability when TAF10 is inhibited in The Cancer Genome Atlas (TCGA) as a large-scale cancer database, it was confirmed that the survival probability was increased (see FIG. 2B). In addition, data analysis was performed to determine a correlation between increased expression of TAF10 and the sternness score required for cancer growth, and a positive correlation between the both was confirmed. The sternness scores were calculated by selecting colorectal cancer stem cell-specific genes (ex, MYC, LGR5, and ASCL2) known in the art, and comparing average expression of genes selected from the colorectal cancer cell lines and TAF10-inhibited colorectal cancer cell lines used in Example of the present invention. Through this, it was confirmed that the sternness scores increased with the expression of TAF10 (see FIG. 2C).

### Example 3. Determination of effects of TAF10 inhibition on WNT signaling pathway, metastatic potential, stemness, and expression of representative genes expressed by normal enterocytes

The effects of TAF10 inhibition on a WNT signaling pathway, a metastatic potential, stemness, and expression of representative genes expressed by normal enterocytes were confirmed, and the results were illustrated in FIG. 3.

As a result, it was confirmed that the expression of genes AXIN2, RAC1, PLCB1, TCF7, CTNNB1, and CCND1 of the WNT signaling pathway was reduced by TAF10 inhibition (FIG. 3A). In addition, it was confirmed that the expression of genes VIM and SMAD4 related to mesenchymal cells was decreased, and the expression of a tumor suppressor gene ATF3 related to metastasis of colorectal cancer was increased (FIG. 3B). On the other hand, it was confirmed that the expression of CD166 (ALCAM), a representative gene of colorectal stemness, was decreased (FIG. 3C), and the expression of KRT19, a representative gene of normal colorectal cells, was increased (FIG. 3D).

### Example 4. Confirmation of effect of TAF10 inhibition on cell growth depending on type of colorectal cancer cell line

Through Example, it was confirmed that the TAF10 inhibition suppressed the growth of HCT116, a colorectal cancer cell line. As a result, the expression genes and proteins related with the growth rate and cell growth and the expression of genes and proteins related to cell cycle arrest were confirmed according to TAF10 inhibition (using siRNA) in HT29 and CACO2 colorectal cancer cell lines, which were other colorectal cancer cell lines. In addition, the cell cycle was confirmed according to TAF10 inhibition, and whether there was an increase in senescent cells was confirmed. The senescent cells were confirmed by staining the senescent cells using a senescence b-galactosidase staining kit (Cell signaling).

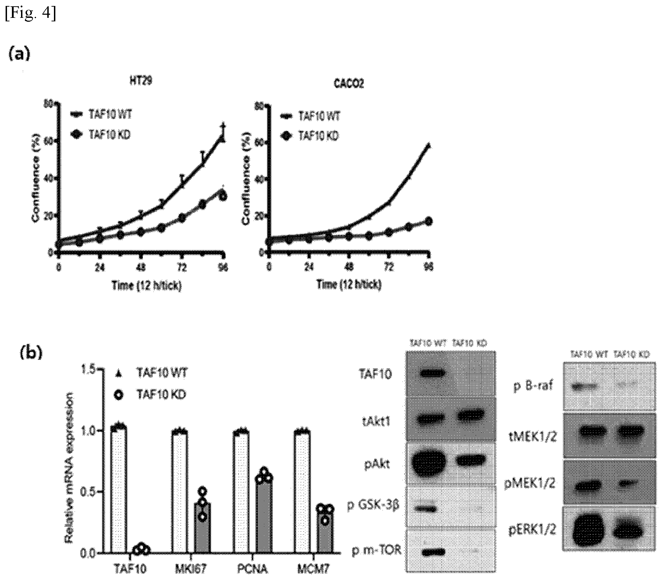

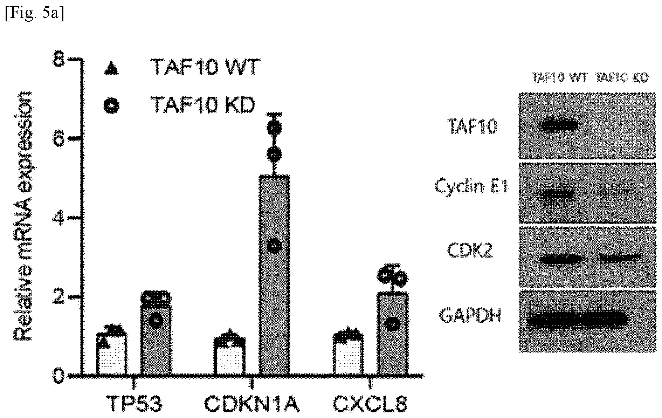

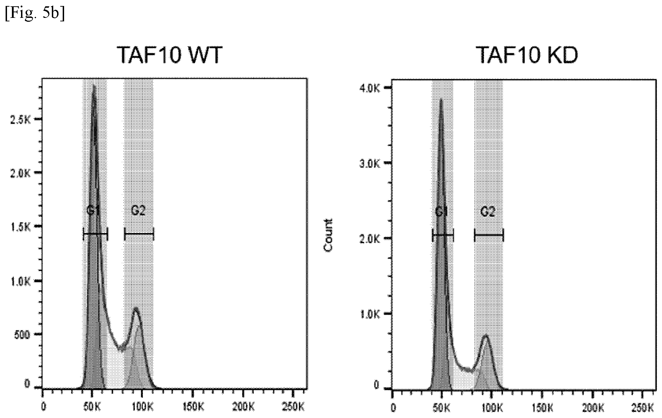

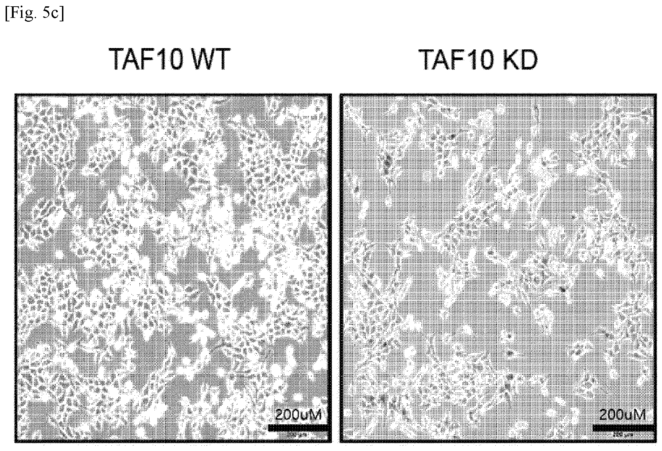

As a result, as illustrated in FIG. 4, it was confirmed that the growth rate of HT29 and CACO2 was also reduced by TAF10 inhibition (FIG. 4A). In addition, in HCT116 where TAF10 was inhibited, the expression of genes MKI167, PCNA, and MCM7 related to cell growth and proteins tAkt1, pAkt, pGSK-3β, m-TOR, pB-raf, tMEK1/2, pMEK1/2, and pERK1/2 was confirmed, and thus, it was confirmed that the expression thereof was reduced by TAF10 inhibition (FIG. 4B). In addition, the expression of genes TP53, CDKN1A, and CXCL8 and proteins Cyclin E1, CDK2, and GAPDH related to cell cycle arrest was confirmed, and thus it was confirmed that the expression thereof was increased by TAF10 inhibition (FIG. 5A). In addition, in the case of HCT116 in which TAF10 was inhibited, it was confirmed that the cell cycle was arrested (FIG. 5B), and thus, it was confirmed that the number of senescent cells was increased (FIG. 5C). Therefore, it was confirmed that TAF10 inhibition regulated the growth and survival of cancer by inhibiting the MEK and AKT pathways.

### Example 5. Confirmation of cancer cell growth inhibition effect in vivo by TAF10 inhibition

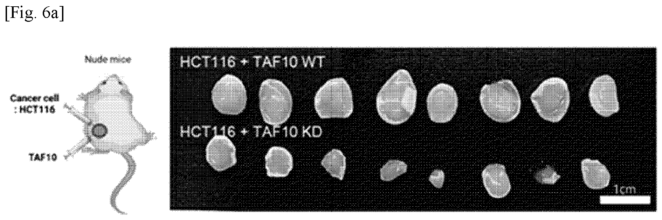

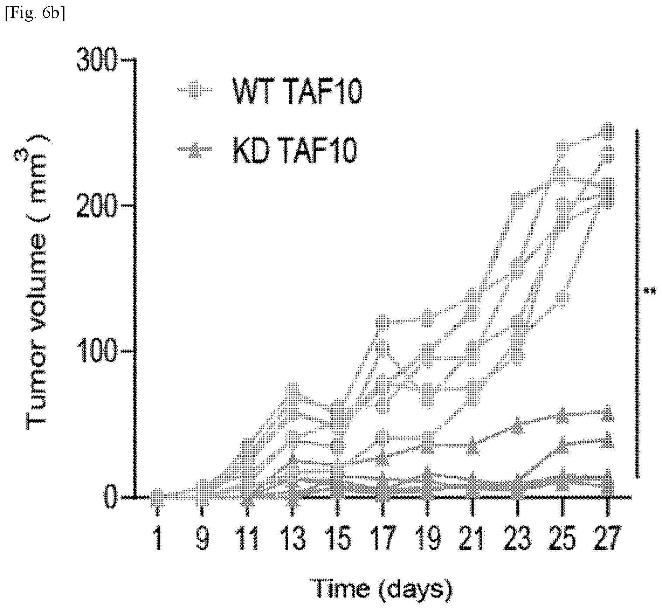

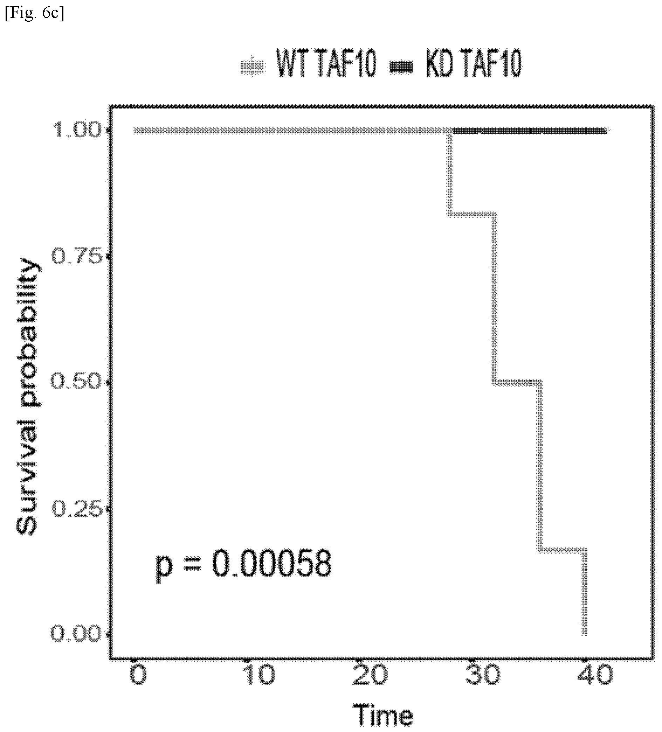

In the colorectal cancer-inducing animal model of Experimental Example 2, the growth of cancer cells was confirmed depending on the presence or absence of TAF10 inhibition. As a result, as illustrated in FIG. 6, it was confirmed that the growth of cancer cells in an experimental group in which TAF10 was inhibited and colorectal cancer was induced was significantly reduced compared to an experimental group in which TAF10 was not inhibited and colorectal cancer was induced (FIGS. 6A and 6B). In addition, it was confirmed that the survival probability of the colorectal cancer-induced animal model in which TAF10 was inhibited was significantly increased (FIG. 6C).

### Example 6. Confirmation of effect of TAF10 inhibition on vascularization in colorectal cancer tissue

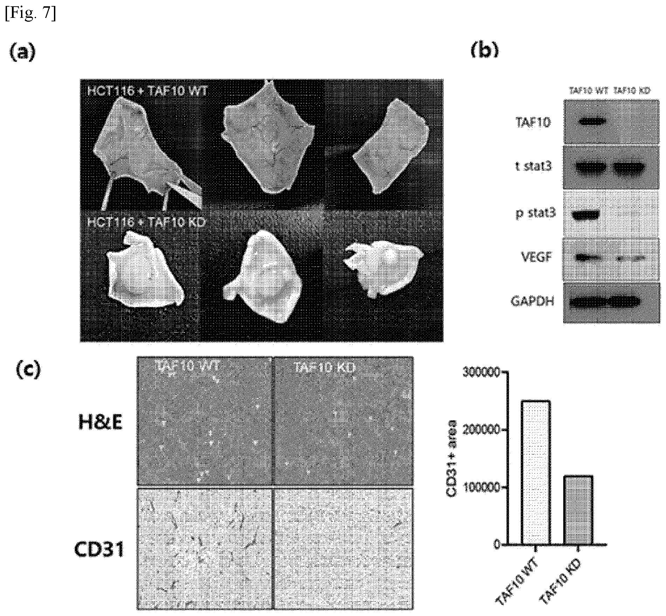

The effect of TAF10 inhibition on vascularization was confirmed. As a result, as illustrated in FIG. 7, it was confirmed that the vascularization was reduced in the colorectal cancer tissue of the experimental group in which TAF10 was inhibited (FIG. 7A). Further, it was confirmed that the vascularization was regulated by the expression of VEGF by a STAT3 pathway (FIG. 7B). In addition, the expression of CD31, a vascular marker, was confirmed through Immunohistochemistry (IHC) (Anti-CD31 antibody; ab182981; Abcam). As a result, it was confirmed that the vascularization was significantly reduced in the experimental group in which TAF10 was inhibited (FIG. 7C), and it could be seen that the TAF10 inhibition suppressed the vascularization.

### Example 7. Confirmation of effect of TAF10 inhibition on differentiation of colorectal cancer and expression of cancer stem cells

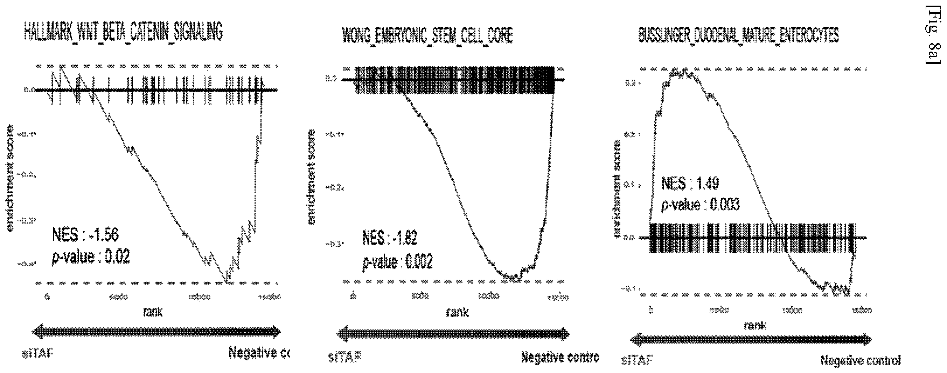

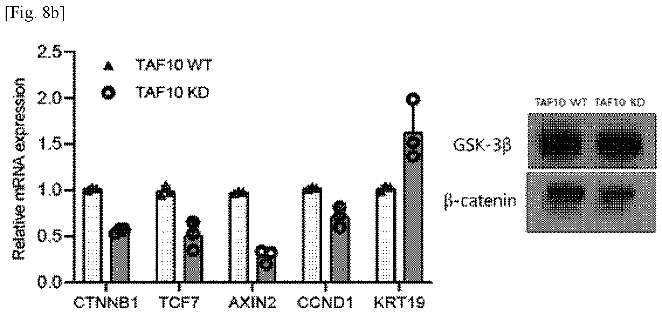

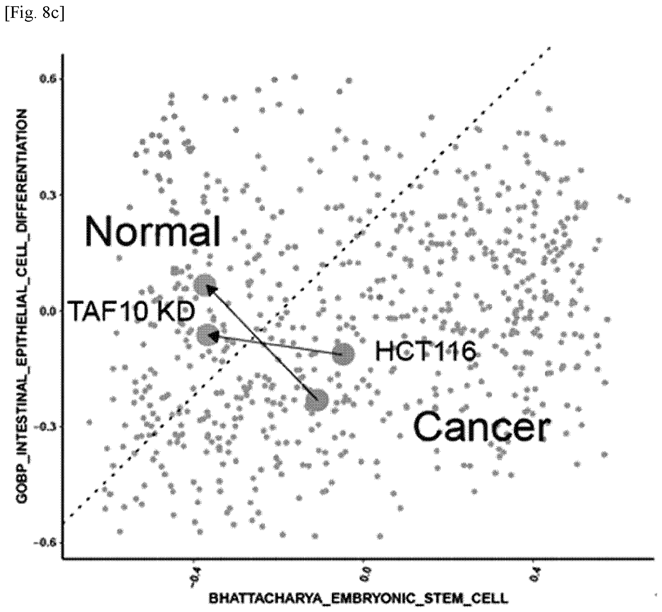

The effects of TAF10 inhibition on differentiation of colorectal cancer and expression of cancer stem cells were confirmed. Transcriptomes when TAF10 was inhibited were obtained through RNA sequencing (preparation of bulk RNA sequencing data), and transcriptomes of colorectal cancer patients and transcriptomes of normal people were obtained from The Cancer Genome Atlas (TCGA), and principal component analysis (PCA) was performed and the results thereof were illustrated in FIG. 8.

More specifically, the expression of a gene set related to a WNT-β-catenin pathway known to be important in the development process of colorectal cancer and the expression of a gene set related to the stem cell scores were confirmed by analyzing bulk RNA sequencing data of HCT16 that suppressed TAF 10 and HCT16 that did not suppress TAF10. As a result, it was confirmed that when TAF10 was inhibited, the expression of the gene set related to the WNT-b-catenin pathway and genes related to stem cell cores were decreased in set units. In addition, it was confirmed that the expression of the gene set related to enterocytes, one of the differentiation types of colorectal cells, was increased (FIG. 8A).

Based on the results confirmed in FIG. 8A, as a result of confirming the expression of genes CTNNB1, TCF7, AXIN2, and CCND1 related to the WNT pathway using qPCR, it was confirmed that the expression was decreased when TAF 10 was inhibited. On the other hand, the expression of KRT19, a differentiation marker, was increased. In addition, through Western blot, it was confirmed that the activities of GSK-3β and β-catenin, major markers of the WNT pathway, were changed when TAF 10 was inhibited (FIG. 8B).

Because the transcriptome represented the state of the cell, reversal was confirmed at the transcriptome level. For this purpose, bulk RNA sequencing data and patient data of TCGA were visualized as sternness scores and differentiation scores in a two-dimensional dot plot. As a result, it was confirmed that the transcriptome level of HCT16, which inhibited TAF 10, moved to the axis of normal cells (FIG. 8C).

Therefore, when TAF10 was inhibited in colorectal cancer, it has been confirmed that the transcriptomes moved to normal cells and the cancer sternness decreased. Thus, it may be seen that TAF10 plays a role in inducing the differentiation of colorectal cancer to increase the expression proportion of cancer stem cells.

### Example 8. Confirmation of synergistic effect between TAF10 inhibition and anticancer agents

Meanwhile, an increase in the cancer sternness of cancer cells may also cause increased resistance of cancer cells to anticancer agents. Therefore, a colorectal cancer animal model in which TAF10 was inhibited or not was treated with the anticancer agent, oxaliplatin (1 µM concentration) and its therapeutic effect was confirmed.

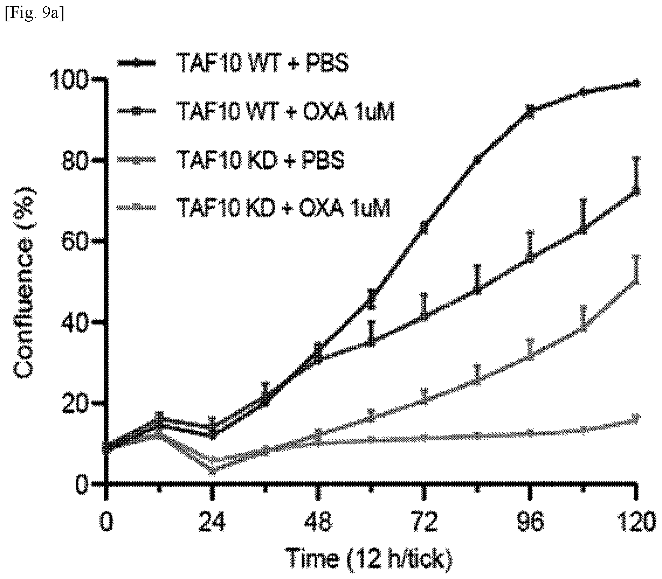

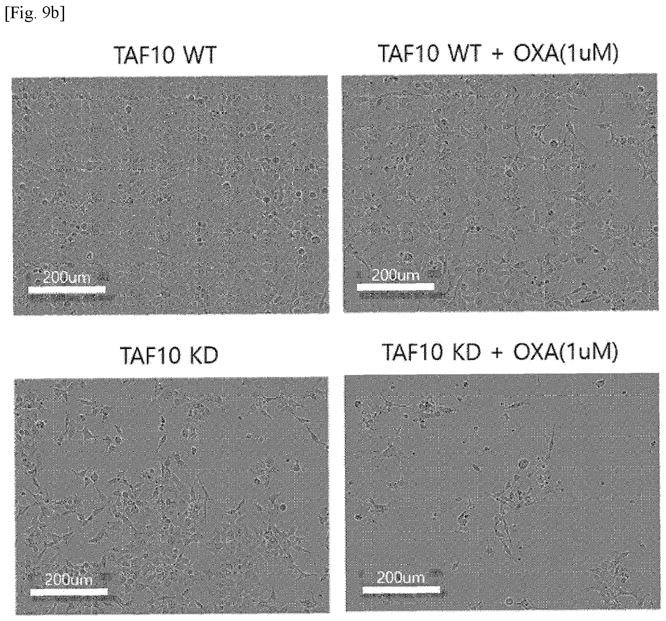

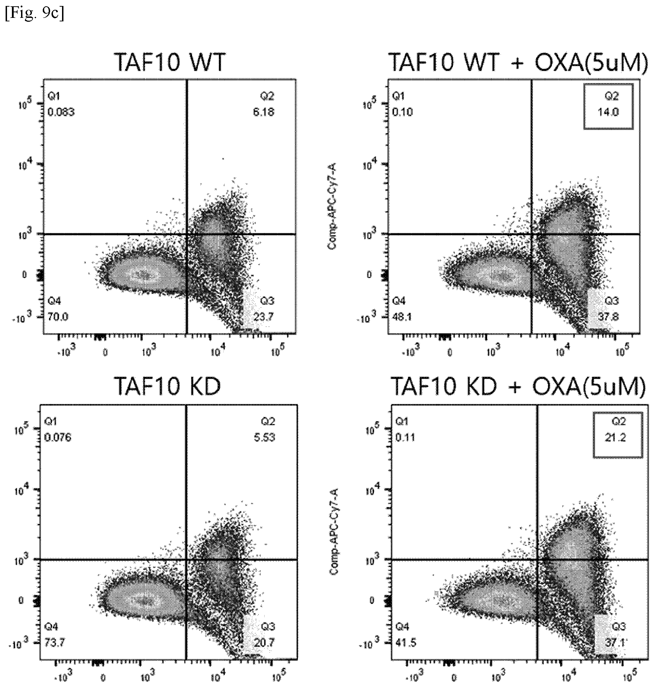

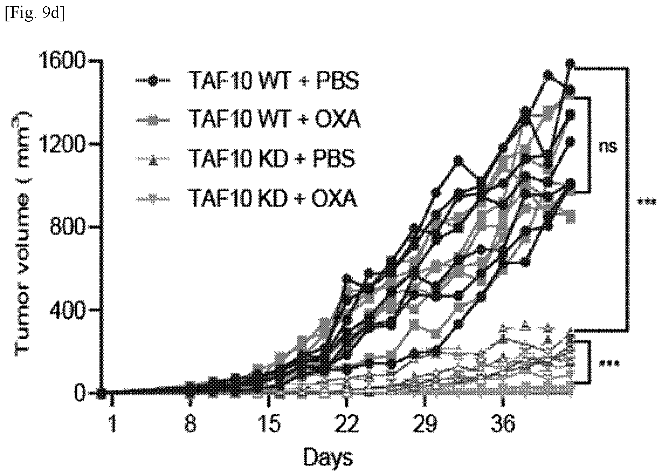

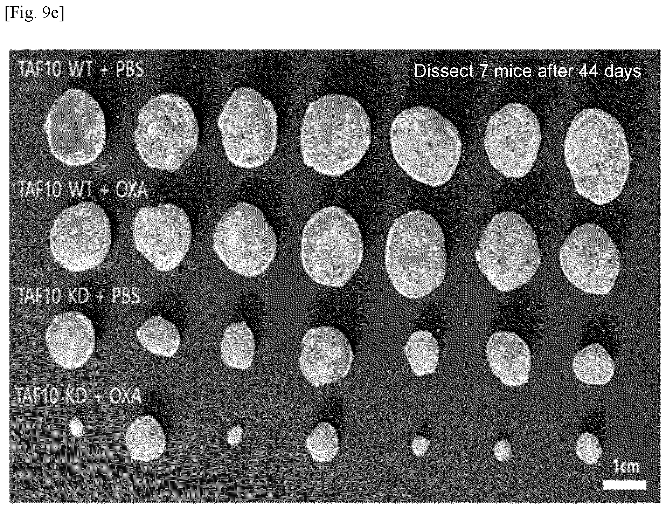

As a result, as illustrated in FIG. 9, it was confirmed that inhibition of TAF10 showed a synergistic effect on the anticancer effect of oxaliplatin. More specifically, in an experimental group where TAF10 was inhibited and oxaliplatin was treated, the cancer confluence was significantly reduced (FIG. 9A). In addition, as a result of staining cells by treating a PI solution capable of confirming cell death, it was confirmed that proliferation was suppressed when TAF 10 was inhibited, and that the number of dying cells increased when treated with oxaliplatin along with TAF10 inhibition (FIG. 9B).

In addition, the degree of cell death through PI and 7-AAD was confirmed through FACS. In this regard, Q1 means NECROSIS, Q2 means LATE APOPTOSIS, Q3 means EARLY APOPTOSIS, and Q4 means live cells. The degree of cell death may be determined by increasing the ratio of Q2. As a result, it was confirmed that the ratio of Q2 increased when TAF10 was inhibited and oxaliplatin was combined, so that it was confirmed that cell death increased (FIG. 9C).

In addition, as a result of confirming the tumor volume over time, it was confirmed that the tumor size was also significantly reduced (FIGS. 9D and 9E).

In addition, the combined effect in vivo was confirmed using a colorectal cancer-inducing animal model prepared by subcutaneously injecting HCT116 in which TAF10 was inhibited or HCT116 in which TAF10 was not inhibited. From two weeks after the tumor in each experimental group was formed, WT and KD were divided into two experimental groups. One experimental group was treated with phosphate buffered saline (PBS), and the other experimental group was treated with oxaliplatin at a concentration of 10 mg/kg once every two days. After breeding for 44 days, the experimental groups were dissected, tumors were obtained, and then each tumor was confirmed. As a result, it was confirmed that the tumor size of the experimental group in which TAF10 was inhibited and oxaliplatin was treated was significantly reduced compared to the other experimental group (FIG. 9E).

Overall, the inhibition of TAF10 was found to lead to suppressing the growth and proliferation of colorectal cancer cells, reducing vascularization, and inhibiting the WNT signaling pathway, and increase the survival probability of patients as analyzed with a large-scale cancer database. In particular, reversal of the cancerization process, which is the differentiation of colorectal cancer cells into normal cells, was confirmed. Unlike conventional anticancer agents that simply induce the death of cancer cells, the present invention may thus be advantageously utilized as a treatment method for converting colorectal cancer cells into normal cells while excluding side effects of normal cell death that may occur during anticancer treatment.

## Claims

1. A pharmaceutical composition for preventing or treating colorectal cancer, comprising a TATA-Box Binding Protein Associated Factor 10 (TAF10) inhibitor.

2. The pharmaceutical composition for preventing or treating colorectal cancer of claim 1, wherein the TAF10 inhibitor is at least one selected from the group consisting of siRNA, shRNA, miRNA, a ribozyme, an antisense nucleic acid, a DNA/RNA chimeric polynucleotide, an antibody, or a vector expressing these, targeting a TAF10 gene or protein.

3. The pharmaceutical composition for preventing or treating colorectal cancer of claim 2, wherein the siRNA includes base sequences represented by SEQ ID NOs: 1 and 2.

4. The pharmaceutical composition for preventing or treating colorectal cancer of claim 2, wherein the shRNA includes a base sequence represented by SEQ ID NO: 7.

5. The pharmaceutical composition for preventing or treating colorectal cancer of claim 1, wherein the TAF 10 inhibitor induces differentiation of colorectal cancer cells into normal cells or normal-like cells.

6. The pharmaceutical composition for preventing or treating colorectal cancer of claim 1, wherein the TAF10 inhibitor suppresses proliferation or growth of colorectal cancer cells.

7. The pharmaceutical composition for preventing or treating colorectal cancer of claim 1, wherein the TAF10 inhibitor suppresses vascularization of colorectal cancer cells.

8. The pharmaceutical composition for preventing or treating colorectal cancer of claim 1, wherein the TAF10 inhibitor accelerates senescence of colorectal cancer cells.

9. A food composition for preventing or alleviating colorectal cancer, comprising a TATA-Box Binding Protein Associated Factor 10 (TAF10) inhibitor.

10. The food composition for preventing or alleviating colorectal cancer of claim 9, wherein the TAF10 inhibitor is at least one selected from the group consisting of siRNA, shRNA, miRNA, a ribozyme, an antisense nucleic acid, a DNA/RNA chimeric polynucleotide, an antibody, or a vector expressing these, targeting a TAF10 gene or protein.

11. The food composition for preventing or alleviating colorectal cancer of claim 10, wherein the siRNA includes base sequences represented by SEQ ID NOs: 1 and 2.

12. The food composition for preventing or alleviating colorectal cancer of claim 10, wherein the shRNA includes a base sequence represented by SEQ ID NO: 7.

13. A pharmaceutical composition for enhancing sensitivity of colorectal cancer cells to anticancer agents, comprising a TATA-Box Binding Protein Associated Factor 10 (TAF10) inhibitor.

14. The pharmaceutical composition for enhancing sensitivity of colorectal cancer cells to anticancer agents of claim 13, wherein the anticancer agent is at least one selected from the group consisting of oxaliplatin, 5-fluorouracil (5-FU), doxorubicin, irinotecan, carboplatin, paclitaxel, gemcitabine and bortezomib.

15. An anticancer adjuvant comprising a TATA-Box Binding Protein Associated Factor 10 (TAF10) inhibitor.

16. A reagent composition for controlling differentiation of colorectal cancer cells into normal cells or normal-like cells, comprising a TATA-Box Binding Protein Associated Factor 10 (TAF10) inhibitor.

17. A method for inducing proliferation suppression or normal differentiation of colorectal cancer cells, comprising treating colorectal cancer cells with the reagent composition according to claim 16.

18. A screening method of agents for treating colorectal cancer comprising:
(a) treating colon cancer cells with a candidate substance;
(b) measuring the expression level of TAF10 in the colon cancer cells treated with the candidate substance; and
(c) screening the candidate substance as a preparation for treating colorectal cancer when the expression level of TAF10 is lower than that of a control group that is not treated with the candidate substance.

19. A method of treating colorectal cancer, comprising administering a TATA-Box Binding Protein Associated Factor 10 (TAF10) inhibitor to a subject.
